# EUROPEAN PATENT APPLICATION

(11) **EP 2 229 959 A2**
(43) Date of publication of application: **22.09.2010**
(21) Application number: 10002845.5
(22) Date of filing: 18.03.2010
(51) Int. Cl.: A61L 2/28, C12M 1/26, C12Q 1/04

(54) **Standarized production of mature biofilms**

(30) Priority: 18.03.2009 CH 4052009
(71) Applicant: EMPA Eidgenössische Materialprüfungs- und Forschungsanstalt, CH-8600 Dübendorf (CH)
(72) Inventor: Fäh. Daniel, 9053 Teufen (CH); Amberg, Caroline, 9055 Bühler (CH); Mauclaire, Laurie, 8008 Zürich (CH); Zinn, Manfred, 9030 Abtwil (CH)
(74) Representative: Rüedi, Regula Béatrice

(57) **Abstract**

The invention provides methods and devices for the standarized production of mature biofilms as well as methods and devices for assessing the biofilm removal efficiency of treatments.

## Description

### Technical Field

The present invention relates to methods and devices for the standardized production of mature biofilms as well as methods and devices for assessing the biofilm removal efficiency of different treatments.

### Background Art

Biofilm is a particular microbial ecosystem in which microorganisms strongly interact with the surface. Expensive strategies to control damage caused by biofilms are undertaken, in particular in such fields as medicine, water treatment and food production. Biofilm removal is considered a challenge in the art. Therefore, several treatment procedures and/or agents have been proposed. In order to evaluate the effectiveness of said procedures and/or agents, the state of the art describes several approaches:

In WO0077162 a model biofilm for efficacy assessment of antimicrobials is described. Said model biofilm is developed during 48 hours on a flat surface under static conditions. Subsequently, the biofilm is dried and further used for determining the efficiency of biocides according to the AOAC Official Method of Analysis. The analysis of the biofilm occurs microscopically after dual staining (exopolymeric substances with alcian blue and bacterial cells with carbol fuchsin) or via culturing the cells.

WO0302877 describes an apparatus and a method for monitoring the ability of automated endoscopic cleaning devices to remove bacterial biofilm in an endoscope. The method for assessing and evaluating a cleaning process comprises the steps of providing a biofilm on a support, subjecting the support and biofilm to the cleaning process to be assessed and evaluated; and evaluating the cleaning process by analyzing the support for the presence of the remaining biofilm. The support is a simulated endoscope, i.e. a long, narrow lumen. The presence and/or amount of residual contaminant in or on the simulated instrument after the cleaning process is used as indicator of the cleaning process.

In WO9941354 a protocol for simulated natural biofilm formation is described. A natural biofilm inoculum is used to form a simulated natural biofilm on retrievable slides in an annular reactor. The monitor protocol in the annular reactor subjects the bacterial consortia to simulated household drain conditions. The simulated natural biofilm on a slide surface can be used to test biocide activity in simulated environmental conditions.

Furthermore, a repeatable laboratory method to grow biofilm in a reactor system that simulates the toilet bowl environment and which could be used for biocide activity testing was described (Pitts et al. (2001): A repeatable laboratory method for testing the efficacy of biocides against toilet bowl biofilms. J. Appl. Microbiol. 91: 110-117). This reactor system incorporates intermittent nutrient flow and simulates the accumulation of bacterial biofilm that occurs in toilet bowls. The reactor can be used to evaluate potential antimicrobial and antifouling treatments to control biofilm formation in toilet bowls, preferably a treatment with chlorine.

However, the proposed systems suffer from various disadvantages.

In the case of WO0077162 the model biofilm poorly represents the biofilm grown in real systems such as washing machines. Furthermore, the invention makes use of standard procedures to investigate biocide efficiency.

Object of WO0302877 is to provide a device that monitors the ability of automated endoscopic cleaning devices to remove bacterial biofilm in an endoscope. Consequently, the device has a long, narrow lumen, wherein a biofilm may develop. Said environment is different to wider open surfaces. The presence and/or amount of residual contaminant in or on the simulated instrument after the cleaning process serves as an indicator of the effectiveness of the cleaning process. The description, however, only discloses the illumination of the biofilm by light in the infrared or ultraviolet range and thus enables a qualitative determination.

In the case of WO9941354 the medium is drained and replaced periodically (every 24 hours). The protocol provides an environment for biofilm growth simulating drain conditions, however the conditions for cell attachment and biofilm growth are not as optimal as in a continuous culture reactor.

Although in the case of Pitts et al., the bioreactor simulates adequately the biofilm grown in real toilet bowl, limitations are that the cultivation is not continuous (as for WO9941354) the formed biofilm is not supposed to be shipped and that tests of cleaning procedures have to be conducted in the bioreactor. Thus, the teaching cannot be adapted to assessing the effectiveness of cleaning procedures by the end-user under real circumstances.

Moreover, technological developments often suffer from problems of reproducibility because of a lack of standardized methods to form and analyze biofilms.

A number of devices have been developed to enable the study of biofilm formation, such as the Robbins device (Kharazmi et al., 1999) and the (laminar) flow cell (Millar et al., 2001). Both systems suffer from the fact that the growth conditions at the entrance of the device significantly differ from the conditions at the exit with respect to nutrition and oxygen partial pressure. The impact of said gradient problem was tried to be reduced by including internal reference coupons (supports) that were used to compare between different materials or antifoulants. However, the amount of biofilm formed on a test surface is not the same for different test coupons and therefore a standarized biofilm cannot be established by these methods. Another significant disadvantage of flow cells is the limited potential for scale-up.

Other devices like the annular tube reactor (Gjaltema et al., 1994) or the rototorque (Peyton et Characklis, 1993) have a higher potential for scale-up but have the following drawbacks: (i) they operate as batch system and thus do not offer the possibility to make continuous cultivation; (ii) the aeration in the reactor is extremely limited and thus does not offer ports for air or the possibility to connect an oxygen loop; (iii) there is no possibility to control and adjust the bioprocess in the reactor (e.g. connection of pH electrode and acid/ base solutions); (iv) they can not be autoclaved in-situ; and (v) the jacket used for temperature control is not as reliable as an internal heating system.

In EP1491505, a method for measuring and controlling the formation of biofilm in a water system is described. For said purpose, a clean metal plate is fixed within the water system. After a certain time, the plate is removed from the water system and biofilm formation is assessed by staining the biofilm in an unspecific manner with safranin. However, the procedure aims at testing the potential of biofilm formation in a water pipe, i.e. the amount of biofilm formed in a certain time frame. It does not provide a method for providing a standardized biofilm.

JP2005278473 describes a method for growing a biofilm providing a plurality of biofilm adherent sites with a surface material, a liquid growth medium flowing across biofilm adherent sites and bacterial inoculums.
However, the model biofilm poorly represents a biofilm grown in real systems such as washing machines.

Hence, there is a need in the art for providing a method and a device for assessing the biofilm removal efficiency of different treatments. Preferably, said method and device are simple to use.

Furthermore, there is a need in the art for providing a method and a device for the generation of standardized biofilms.

### Disclosure of the Invention

Hence, it is a general object of the invention to provide a method and a kit for determining the efficiency of a biofilm removal treatment.

Moreover, it is a general object of the invention to provide a method and means for generating standardized biofilms.

In a first aspect, the invention provides a method for determining the efficiency of a biofilm removal treatment, comprising the steps of:
(a) providing one or more standardized mature biofilm(s) on one or more support(s);
(b) subjecting the support(s) and the biofilm(s) to a treatment to be assessed and evaluated; and
(c) determining the degree of removal of the biofilm(s) from the support(s) by comparing to a untreated biofilm(s).

The biofilms used in the described method may be monospecific biofilms or plurispecific biofilms.

In another aspect, the invention provides a kit for determining the efficiency of biofilm removal. Said kit comprises a sample device comprising a carrier which in turn comprises one or more supports with a mature biofilm.

Moreover, the kit may further comprise an evaluation device, such as agents for staining the biofilm and/or extrapolymeric substances. Preferably, a reference biofilm and/or a color scale are present.

Preferably, said kit is used for the method for determining the efficiency of biofilm removal disclosed herein.

In a preferred embodiment, said method and kit as described herein are such that anyone can use the kit without the need of sophisticated instrumental equipment. This is particularly the case when staining methods are used for determinig the degree of biofilm removal.

The method and kit disclosed herein provide a semi-quantitative determination of biofilm removal efficiency and have the advantage that they are easy to handle by the customer himself. The kit can be equipped such that all materials and agents necessary to conduct the test can be handled by the customer himself, in particular when the method and/or the kit are based on biofilm detection by direct staining. Additionally or optionally, a further quantitative analysis can be made when the kit is sent to a laboratory where a standardized test series for biofilm characterization and quantification can be made.

The present invention is e.g. applicable in the fields of medicine, water treatment, cleaning, and/or food production. Furthermore, compositions, biocides, disinfectants and antibiotics can be tested with the method/and or kit as described herein. For example, the invention can be applied to the estimation of biofilm removal in washing machines or other systems (e.g. dishwasher, air conditioner, fridge, cooling tower, paper industry), or the treatment of drinking and waste water pipes. Most advantageously, the present invention is applied in washing performance testing and is thus of interest for washing machine manufacturers, washing detergent manufacturers and/or manufacturers of washing detergents.

As the method and the kit for determining the efficiency of biofilm removal disclosed herein rely on comparison to a positive control, i.e. an untreated biofilm, it is necessary that the biofilms are homogenous and/or standardized so that a comparison and a meaningful deduction of the results can be made.

Therefore, in still another aspect, the invention provides means for the production of biofilms such as a fixing rail for receiving individual supports for biofilm formation which can be interchangeably fixed in the vessel of a bioreactor, preferably in a tank reactor.

Moreover, a supporting body for fixing the fixing rail described herein is provided which allows fixing in an interchangeable manner said rail in a defined position within the vessel of a bioreactor. In a preferred embodiment, said supporting body has a cylinder structure and can rotate at controlled speed.

Furthermore, a bioreactor is provided equipped with said supporting body. The design of the bioreactor is such that one or more fixing rails carrying supports for biofilm formation can be introduced and/or removed from the vessel. Preferably, the bioreactor is equipped with or connected to further means so that the culture is fed continuously. Preferably, the bioreactor is equipped with or connected to further means so that the culture is oxygenized via an oxygen loop.

In a further aspect, the invention provides a method for providing standardized mature biofilm of microorganisms on a support comprising the steps of
(a) providing one or more supports;
(b) inserting the supports into a bioreactor, preferably via a fixing rail as described herein and in the bioreactor described herein;
(c) cultivating the microorganisms; and
(d) harvesting the biofilm formed on the supports,
wherein the culture is oxygenized through a oxygen loop.

It has surprisingly been found that the aeration of the culture via an oxygen loop is advantageous for a standardized biofilm formation. The method and the equipment disclosed herein, i.e. the fixing rail, the supporting body and/or the bioreactor, are preferably used for the production of biofilms which can be used in the kit and method for determining the efficiency of biofilm removal described herein.

### Brief Description of the Drawings

The invention will be better understood and objects other than those set forth above will become apparent when consideration is given to the following detailed description thereof. Such description makes reference to the annexed drawings, wherein:

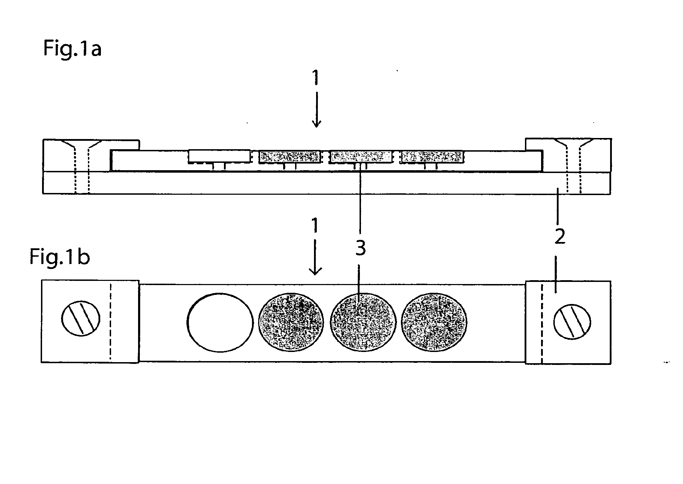

Fig. 1 shows an exemplary sample device 1 comprising elongated carrier structures 2 holding a slide with place for four supports 3, three of them with a mature biofilm 4 and one blank. Fig. 1a is a side view of the sample device 1 and Fig. 1b a top view thereof.

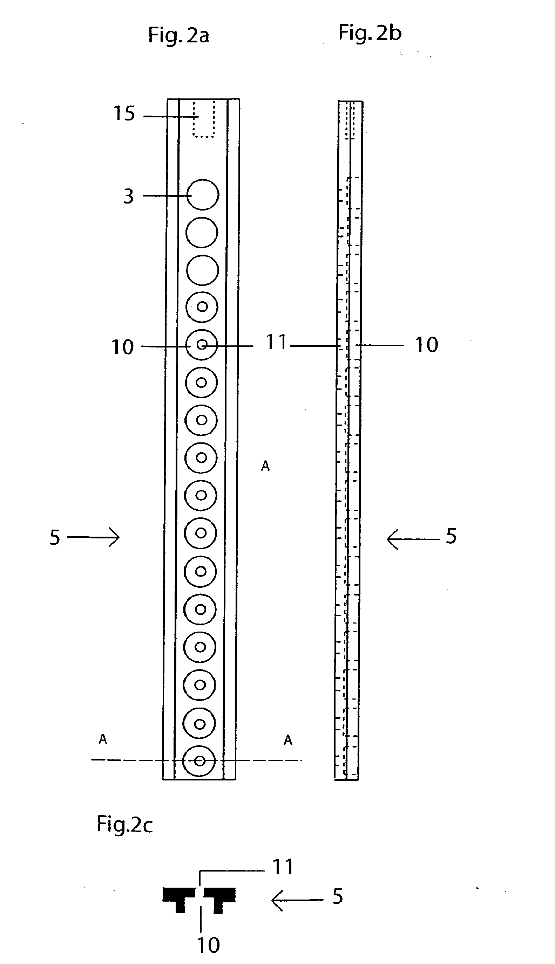

Fig. 2a depicts a front view of an exemplary fixing rail 5 with 16 cavities 10. In the upper three cavities, supports 3 are present. At its upper end, the fixing rail has an aperture 15 for handling the fixing rail 5. Fig. 2b is a cross-sectional side view of the fixing rail 5 which shows the cavities 10 and apertures 11 for applying a releasing means to release the supports 3. An exemplary arrangement of a cavity 10 and aperture 11 can be seen in the cross-sectional top view along the line A-A in Fig. 2c.

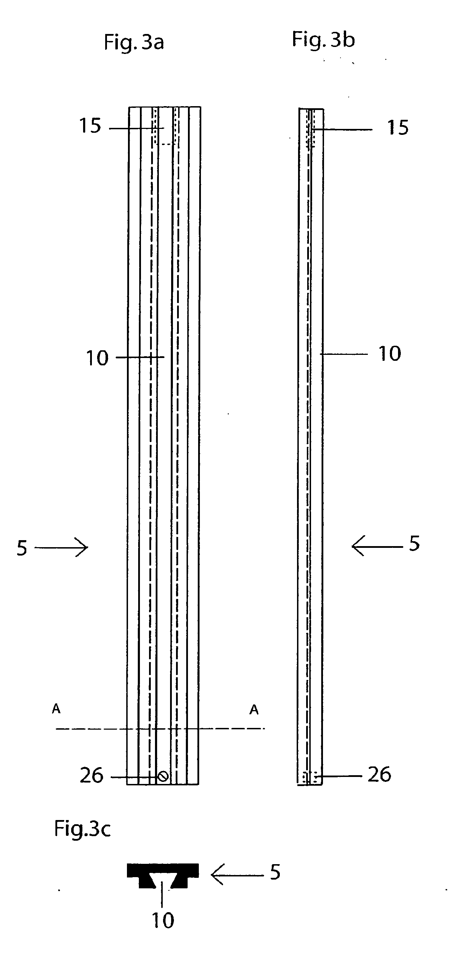

Fig. 3a depicts a front view of an exemplary fixing rail 5 with place for 14 supports. At its upper end, the fixing rail has an aperture 15 for handling the fixing rail 5. At its lower end, the fixing rail 5 has a screw 26 to maintain the supports 3 in place. Fig. 3b is a cross-sectional side view of the fixing rail 5 which shows the cavities 10 where support 3 are inserted. An exemplary arrangement of a cavity 10 can be seen in the cross-sectional top view along the line A-A in Fig. 3c.

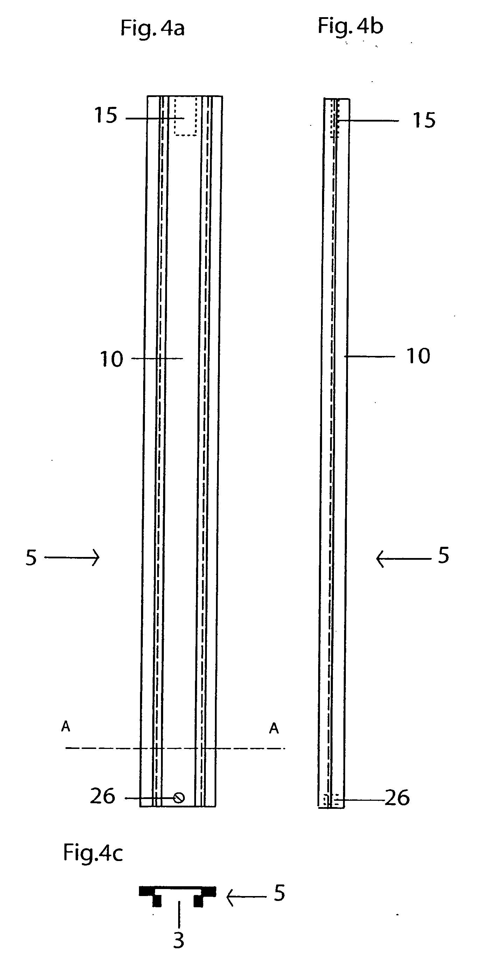

Fig. 4a depicts a front view of an exemplary fixing rail 5 with place for 18 supports. At its upper end, the fixing rail has an aperture 15 for handling the fixing rail 5. At its lower end, the fixing rail 5 has a screw 26 to maintain the supports 3 in place. Fig. 4b is a cross-sectional side view of the fixing rail 5 which shows the cavities 10. An exemplary arrangement of a cavity 10 can be seen in the cross-sectional top view along the line A-A in Fig. 4c.

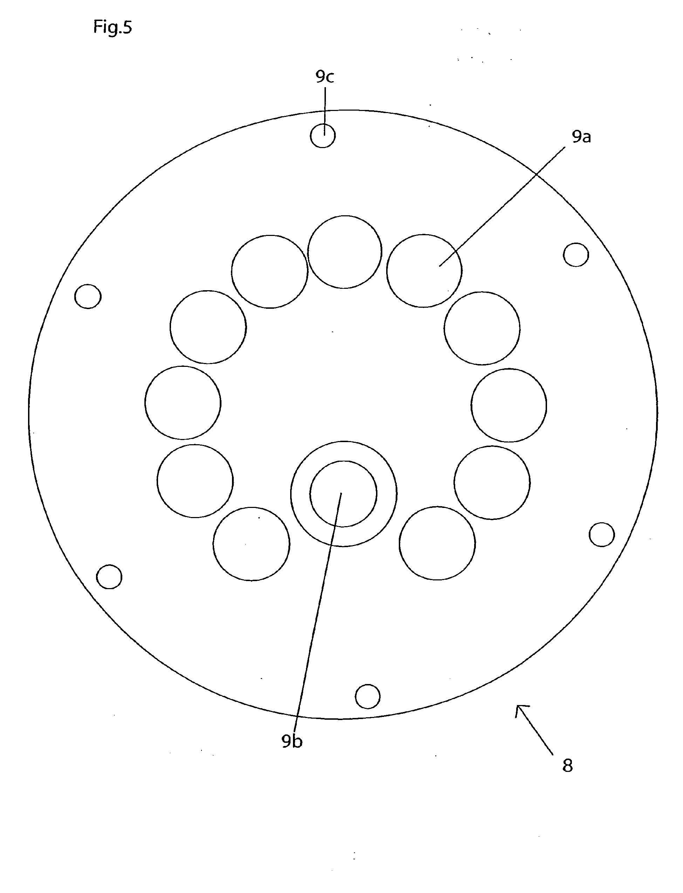

Fig. 5 shows a faceplate 8 of a bioreactor 6 with different apertures 9: Apertures 9a are used for connecting the bioreactor 6 to the external environment (e.g. oxygen loop; medium inlet; waste outlet; acid/base for pH control); apertures 9b are used to insert and remove fixing rails 5; apertures 9c are used to screw the faceplate 8 to the top of the bioreactor 6.

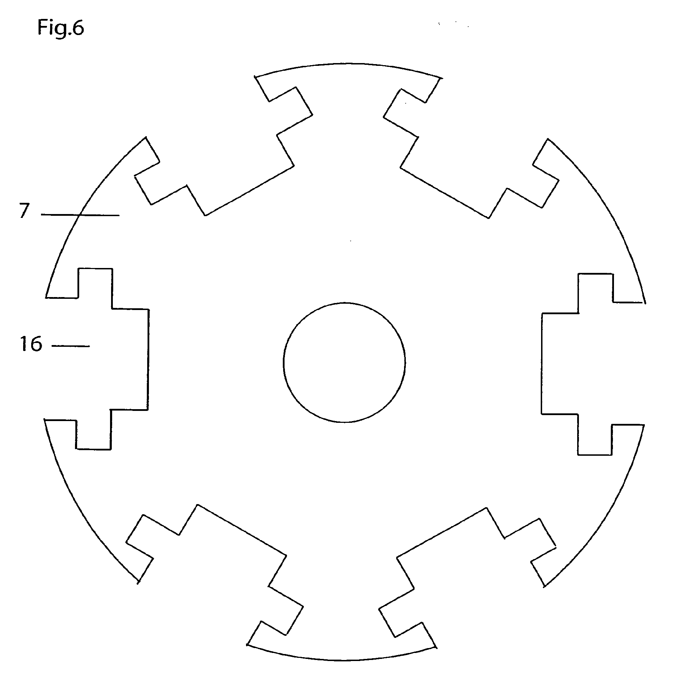

Fig. 6 depicts a topview of an exemplary supporting body 7 having cavities 16 to receive the fixing rails 5.

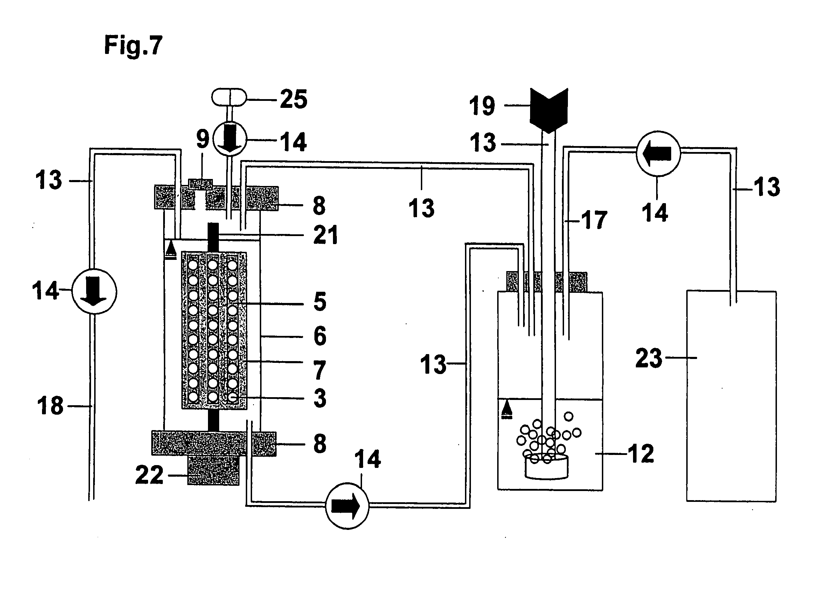

Fig. 7 schematically illustrates a bioreactor 6 with fixing rails 5 mounted on a supporting body 7 and means for an oxygen loop, i.e. an aeration bottle 12, a tank with compressed air 19, pumps 14 and hoses 13. The supporting body 7 is cylinder shaped and can rotate. For said purpose, the supporting body 7 is connected via the central axis 21 to a motor 22. In the illustration, three fixing rails 5 as shown in Fig. 2 can be seen. In this embodiment, the bioreactor 6 is equipped for continuous cultivation. A medium bag 23 is connected via a hose 13 and a medium inlet 17 to the aeration bottle 12. Within said aeration bottle 12, fresh medium is mixed with the culture broth from the bioreactor 6. Via a hose 13, the mixed and aerated liquid is reintroduced into the bioreactor 6. For ensuring continuous cultivation, a medium outlet 18 is provided, as well as an inlet 25 for acid and base to control the pH.

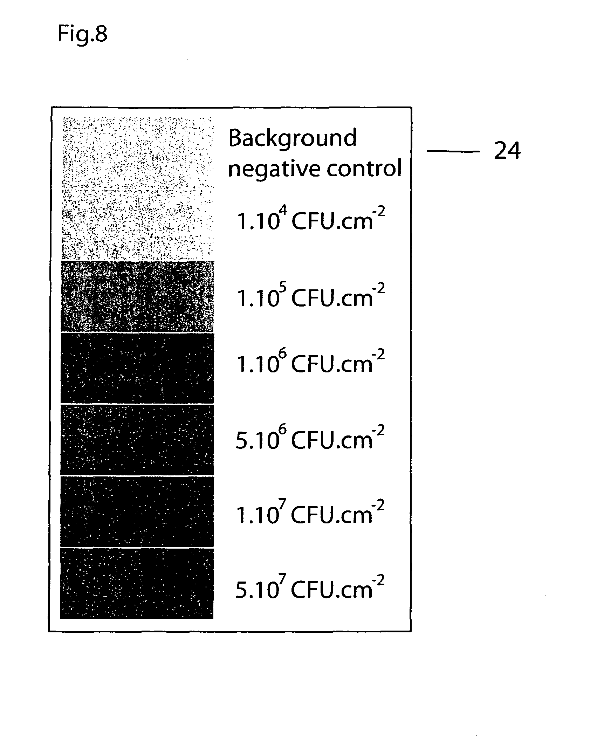

Fig. 8 shows an exemplary color scale.

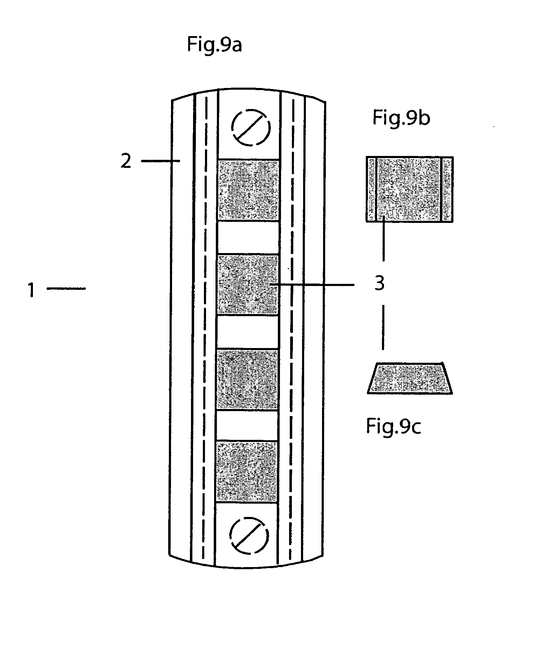

Fig. 9 shows an exemplary sample device 1 comprising elongated carrier structures 2 holding a place for four supports 3, three of them with a mature biofilm 4 and one blank. Fig. 9b is a top view of one support 3 and Fig. 9c is a side view of one support 3.

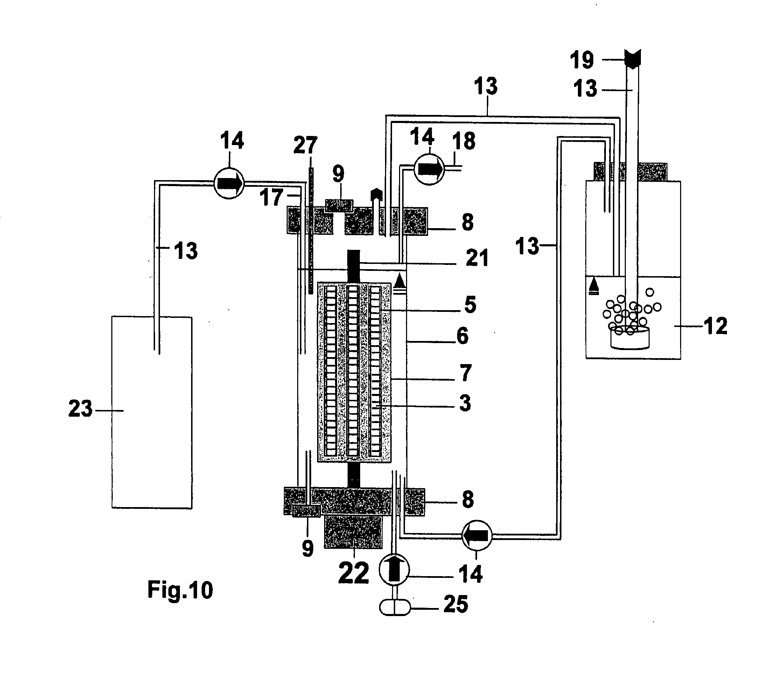

Fig. 10 schematically illustrates a bioreactor 6 with fixing rails 5 mounted on a supporting body 7 and means for an oxygen loop, i.e. an aeration bottle 12, a tank with compressed air 19, pumps 14 and hoses 13.

### Modes for Carrying Out the Invention

The present invention will be described in more detail below. It is understood that the various embodiments, preferences and ranges may be combined at will. Further, depending on the specific embodiment, selected definitions, embodiments or ranges may not apply.

It is noted that the dimensions, configurations and proportional relationships illustrated in the figures of the present application are provided as examples and are not intended to be limiting. Therefore, it is contemplated that the dimensions, configurations and proportional relationships of the present invention may vary from those illustrated in the figures.

In a first aspect, the invention provides a method for determining the efficiency of a biofilm removal treatment comprising:
(a) providing one or more standardized mature biofilm(s) on at least one support ;
(b) subjecting the support(s) and the biofilm(s) to a treatment to be assessed and evaluated; and
(c) determining the degree of removal of the biofilm(s) from the support(s) by comparing to an untreated biofilm(s).

The biofilm(s) typically consist of microorganisms of a single species, i.e. monospecific biofilms are provided, or of several species, i.e. plurispecific biofilms.

As used herein, the term "biofilm" or "mature biofilm" refers to associated and/or accumulated and/or aggregated microbial cells, their products (e.g. exopolymeric substances)and inorganic particles adherent to a living or inert surface. Preferably, the biofilm is the biofilm described herein, more preferably obtainable by the methods described herein.

Typically, the biofilms are present on a support 3, also termed "coupons" or "chips" in the art. Thus, the supports 3 are structures offer a surface for the microorganisms to attach on and to build a biofilm. The supports 3 are preferably made of a sterilizable material and may have different sizes and dimensions. Exemplary supports 3 are depicted in Fig. 1b, Fig. 2a and Fig. 9. These supports 3 are typically present on a carrier structure 2 which can be easily handled by the user. The supports 3 and/or the carrier structure 2 offer the possibility of storage and transport of the biofilms.

The microorganisms which form the biofilm(s) used in the method disclosed herein are typically selected from one or more species selected from the group consisting of gram-positive bacteria, gram-negative bacteria, fungi and algae.

In a preferred embodiment of the invention, at least one microorganism species is selected from the group consisting of Pseudomonas, Staphylococcus, Mycobacterium, Micrococcus, Rhodococcus, Cellulosimicrobium, Microbacterium, Williamsia, Enterobacteriacae (e.g. Escherichia, Klebsiella), Streptococcus, Enterococcus, Leptospira, Clostridium, Listeria, Legionella, Salmonella, Campylobacter, Citrobacter, Shewanella, Bulkholderia, Serratia, Comamonas, Crytptococcus, Rhodotorula, Candida, Saccharomyces, Penicillium and Cladosporium species.

Preferably, a set of several different biofilms is used, i.e. a number of supports 3, each with a monospecific biofilm. Any mix of species is possible. More preferably, each support 3 of one set carries a biofilm of a different group of microorganism, i.e. one support with a biofilm of one species of gram-positive bacteria, one support with a biofilm of one species of gram-negative bacteria, one support with a biofilm of a fungal species and/or one support with a biofilm of an algal species.

The term "treatment" as used herein refers to washing, cleaning, disinfecting, sterilizing or combinations thereof. For example, "washing" applies to a treatment e.g. performed in a washing machine, in a device such as a bowl or in a dish washer. Consequently, the treatment "washing" may well refer to the evaluation of biofilm removal efficiency of chemical agents and/or to the evaluation of biofilm removal efficiency of mechanical forces, e.g. scrapping or the movement of water within the inner basket or the water pipe of the washing machine or within a dish washer. Thus, "removal" refers to biocidal activity as well as to physical removal of microorganisms.

Typically, the biofilms are tested in an environment being different to their production environment, i.e. the biofilms are moved to another environment.

Efforts to identify the species composition of biofilm growing *in situ* in a household washing machine have been undertaken. Most prominent fungal species are Penicillium and Aspergillus spp., predominant bacteria Pseudomonas spp., and a not further identified gram positive rod. In case the method disclosed herein is performed to evaluate the biofilm removal efficiency in a washing machine, preferably several monospecific biofilm are used, preferably consisting of microorganisms selected from the group consisting of Pseudomonas, Staphylococcus, Mycobacterium, Micrococcus, Rhodococcus, Cellulosimicrobium, Microbacterium, Williamsia, Enterobacteriacae(e.g. Escherichia, Klebsiella), Citrobacter, Shewanella, Bulkholderia, Serratia, Comamonas, Crytptococcus, Rhodotorula, Candida, Saccharomyces, Penicillium and Cladosporium species.

In another preferred embodiment, one or more plurispecific biofilms of the mentioned species are used.

The biofilm(s) is placed into the container
wherein the treatment is performed, e.g. - without being limited to - a washing machine, a dish washer, an autoclave or a beaker, and will be exposed to the treatment. In case of a washing machine, the supports are preferably placed close to the water oulet pipe and where applicable, between the inner and outer drum of a washing machine.

After being exposed to the treatment, the biofilm or set of biofilms will be evaluated for the degree of removal of the biofilm. Said determination is done by comparing the treated biofilm or set of biofilms with an untreated (positive) control. For said purpose, a second biofilm or set of biofilms will be provided which will not undergo the treatment and serves as positive control. This second biofilm or set of biofilms will preferably be taken from the same production batch as the first set, in particular because it underwent identical storage and shipping conditions.

In a much preferred embodiment of the present invention, the determination of the degree of removal, i.e. step (c), is semi-quantitative or quantitative, preferably by visual detection, in situ detection or indirect detection. Suitable determination methods for the biofilm are (without being limited to):
(i) visual detection, e.g. through staining of microbial cells with crystal violet, safranine or carbol fuchsin; or staining of exopolymeric substances (EPS), e.g. by alcian blue or other methods known to the skilled person;
(ii) *in situ* detection methods by staining and microscopic analysis of microbial cells such as DAPI, SYTO or SYBRII; or *in situ* detection of EPS via staining by alcian blue or ruthenium red or calcofluor white; or *in situ* detection of microbial activity via staining by tretazolium salts or detection of pH changes or other methods known to the skilled person;
(iii) indirect detection of microbial cells after detachment of the biofilm from the support 3 by e.g. cultivation, PCR, RT-PCR or flow cytometry; or indirect detection of microbial activity by e.g. detection of pH changes or other methods known to a skilled person.

As used herein, "staining the biofilm" refers to staining of the cells and/or to staining of exopolymeric substances.

The visual detection methods have the advantage that they can be applied by the end-user and an evaluation of a treatment can be done without the need of elaborate instrumentation. Additionally or alternatively, the treated and un-treated samples may be sent by the end-user to a test laboratory, where different procedures, such as those mentioned under (ii) and (iii), are performed.

In case the biofilm or set of biofilms are stained, the method preferably involves comparing the treated and the un-treated biofilm(s) with a color scale. The color scale indicates the staining intensity for different biofilm densities. Positive and negative controls are used to adjust the reading of the staining intensities in case that the biofilms get damaged during transport (positive-control) or treatment discolors or otherwise affects the support (negative-control) (see an exemplary color chart in Fig.8). Color charts 24 are preferably provided for each type of biofilm 4 and each type of support 3.

To use the color scale 24, one should first estimate the staining intensity of the positive control (i.e. coupons 3 with a mature biofilm which were not treated) and compare the positive control with the staining intensity of the coupons 3 with a mature biofilm having undergone treatment such as a washing process. Because the color scale 24 preferably indicates the equivalent to colony forming units (CFU) (see the embodiment shown in Fig. 8), one can rapidly quantitatively estimate the efficacy of the treatment by calculating log reduction (e.g. 1 log reduction = 90% killed; 2 log reduction = 99% killed). The determined efficacy may be corrected by means of a negative control.

As the support on which the biofilm is provided may suffer from discoloration or staining, in a preferred embodiment, one or more supports without biofilm are preferably used as negative control. Said blank support is exposed to the treatment in the same manner as those supports carrying a biofilm and serves as negative control for any changes that occur to the support due to the exposition of different mechanical and/or chemical forces during the treatment. Naturally, any number of supports carrying a biofilm and more than one blank support may be present. The upper part of the exemplary color scale 24 as shown in Fig. 8 indicates the background color after staining of the coupon 3 which is used as negative control.

It is necessary to provide homogenous mature biofilms in a standardized manner so that the comparison of step (c) leads to a meaningful result. Contrary to the known state of the art methods, with the methods and equipment disclosed herein the skilled person is able to produce mature biofilm with a defined (or controlled) strain composition in a repeatable and/or reproducible manner. For example, the biofilms produced with the methods and equipment disclosed herein have 3D structure with a same thickness, preferably at least 10 µm, and/or the same amount of exopolymeric substances, preferably containing at least 50µg per cm² polysaccharides and 50µg per cm² proteins, and/or the same cell density and/or the same viability, preferably at least 10⁷ colony forming units per cm². The ratio of polysaccharides (in µg) / living cells is preferably lower than 10⁻⁵, more preferably lower than 10⁻⁶, 10⁻⁷, 10⁻⁸ or 10⁻⁹. As the biofilms are repeatable and reproducible, this applies not only to biofilms stemming from the same production batch, but also to biofilms from a different production batch.

"Repeatable" as used herein refers to the fact that biofilms with little variability can be produced by the same person under the same conditions, whereas "reproducible" as used herein means that the biofilms can be accurately reproduced through the methods and equipment disclosed herein by a different person in a different laboratory.

In case the biofilm is plurispecific, the biofilms further have the same strain composition. In a much preferred embodiment, the biofilms are homogenous in the sense that all biofilms of one production batch or different production batches have the same thickness and the same amount of exopolymeric substance and the same cell density and the same viability.

Therefore, the term "standardized" as used herein refers to a homogenous biofilm as described above, preferably to a biofilm having a controlled (in the sense of a specific) strain composition. Typically, the skilled person would compare several supports with a biofilm in order to determine whether the biofilms are standardized and/or homogenous.

In a preferred embodiment, a biofilm as disclosed herein has a thickness of at least 10 µm and/or at least 50µg per cm² polysaccharides and/or 50µg per cm² proteins and/or at least 10⁷ colony forming units per cm². The ratio of polysaccharides (in µg) / living cells in a biofilm as disclosed herein is preferably lower than 10⁻⁵, more preferably lower than 10⁻⁶, 10⁻⁷, 10⁻⁸ or 10⁻⁹.

In contrast, the prior art cited above does not provide such homogenous mature biofilms in a standardized manner. The term "mature" as used herein preferably refers to a microbial community having developed a significant network of matrix components and 3D structures (e.g. mushroom or pilar-like). The 3D organization of the extracellular matrix (e.g. water channel, mineral crust) holds important implications for biofilm physiology in vivo. Mature biofilm are at steady state (i.e. stable over time). Duration to reach maturity depends on the microbial community and external factors (e.g. nutrients, shear force, support); typically, it takes one to a few weeks to establish a mature biofilm. Attached cells grow first in 2D (in close contact with the support)to form a young biofilm and subsequently grow vertically (3D) and become mature. Consequently, mature biofilms are distinct from young biofilms which are produced in a few hours or days (e.g. produced during 6 hours to 3 days) under sub-optimal growth conditions.

Therefore, the methods for determining the efficiency of biofilm removal disclosed in the prior art differ from the invention disclosed herein. For example, in WO0077162 the biofilms are grown on a porous growth media such as filter paper placed on a nutritive source. The biofilms are grown over a short time, preferably only for 2 to 3 days which is not enough to produce a mature biofilm. Moreover, the mode of alimentation does not allow for growing of a mature biofilm, as the solid growth medium will be quickly depleted and does not ensure a constant and homogenous nutrition of the biofilm.

In case of Pitts et al. (J Appl Microbiol. 2001, (91), p. 110-117), a toilet bowl environment is simulated which is why medium is cyclically removed and fresh medium is added. Consequently, cells which are not resistant to said cyclic fill-and-draw conditions will detach. The strain composition of the biofilm can therefore not be controlled and the proportion of strains within the biofilm can change over time. No pH or oxygen is controlled and hence, the growth conditions are not constant over time; furthermore, the reactor is open to the air. Due to all of the above, the biofilms of Pitts are not standardized. As the method for biofilm removal treatment as described by Pitts is performed within the vessel where the biofilms are grown, the control of an untreated biofilm occurs in a different vessel and is not comparable to the treated one.

Concerning WO03/028772 the culture is grown under conditions in which a liquid bacterial culture grows across a stationary surface. As outlined above, due to a gradient problem, the biofilm cannot be considered as being standardized in the sense of the present invention.

With regard to WO99/41354 the inoculum is extracted from a household drain. The strain composition is not defined and may vary from batch to batch. Also in this case, medium is cyclically removed and fresh medium is added. Cells which are not resistant to said cyclic fill-and-draw conditions will detach and the strain composition of the biofilm can therefore not be controlled.

In a second aspect, the invention provides a kit for determining the efficiency of a biofilm removal treatment comprising a sample device 1 comprising a carrier 2, said carrier 2 comprising one or more supports 3 with a mature standardized biofilm 4. An exemplary embodiment is depicted in Fig. 1.

The kit is particularly suitable for performing the method disclosed above, in particular for the evaluation of the efficiency of biofilm removal treatments, be it biocidal or physical microorganism removal.

The carrier 2 can be made of any suitable material, but is preferably made of a metal or a thermoplastic, such as polypropylene (PP). Preferably, said carrier 2 comprises means for fixing the carrier 2 within a device wherein the treatment for biofilm removal is to be effected such as a washing machine, a dishwasher or a toilet bowl. The carrier 2 comprises one or more supports 3 with a mature biofilm.

The supports 3, in the art also known as "coupons" or "chips", are made of any suitable material such as glass, metal or a thermoplastic, preferably of teflon or polystyrene, more preferably of polypropylene (PP). The surface of the support 2 may be treated in order to increase biofilm 4 adhesion thereon, e.g. by applying a microstructure or by conditioning the surface with biopolymers such as proteins or sugars.

Preferably, the kit further comprises storage and/or transporting means for the carrier 2 and the supports 3 so that they can be shipped to the end-user and eventually from the end-user to a laboratory.

In a preferred embodiment, the kit further comprises a reference biofilm (positive control) and/or an evaluation device. Said evaluation device preferably comprises agents for staining the biofilm. Additionally, a color scale may be present to which the stained biofilm(s) can be compared and the degree of biofilm removal after the treatment may be determined.

In a preferred embodiment, further to one or more supports 3 with a mature standardized and/or homogenous biofilm, consisting either of microorganisms of a single species or of several species, the carrier 2 comprises a support 3 without a biofilm. Said blank support 3 serves as negative control, in particular if the support material is prone to discolor or to be stained through the treatment or any other changes that occur to the support 3 due to the exposition of different mechanical and/or chemical forces during the treatment. Naturally, any number of supports 3 carrying a biofilm and more than one blank may be present.

In one embodiment, the sample device 1 as depicted in Fig. 1 is an elongated carrier structure 2. In a preferred embodiment and as shown in Fig. 1, four supports 3 are present on the carrier structure 2, three of them carrying a biofilm 4 and the forth one being a blank serving as negative control. Naturally, any number of supports 3 can be used and supports 3 can be made of different materials.

The kit preferably comprises several biofilms 4, i.e. a set of biofilms, each biofilm 4 on a different carrier 3.The biofilm is preferably the biofilm described herein, more preferably obtainable by the methods described herein. Preferably, each biofilm 4 consists of a single species or of several species of microorganisms. Preferably, the microorganisms are selected from the group consisting of gram-positive bacteria, gram-negative bacteria, fungi and/or algae. More preferably, each biofilm 4 is a monospecific biofilm. Even more preferably, each carrier comprises an isolate from a different group of microorganism, i.e. one biofilm of gram-positive bacteria, one biofilm of gram-negative bacteria, one fungal biofilm and/or one algal biofilm.

In a preferred embodiment, the microorganisms are selected from the group consisting of Pseudomonas, Staphylococcus, Mycobacterium, Micrococcus, Rhodococcus, Cellulosimicrobium, Microbacterium, Williamsia, Enterobacteriacae (preferably Escherichia or Klebsiella), Streptococcus, Enterococcus, Leptospira, Clostridium, Listeria, Legionella, Salmonella, Campylobacter, Citrobacter, Shewanella, Bulkholderia, Serratia, Comamonas, Crytptococcus, Rhodotorula, Candida, Saccharomyces, Penicillium and Cladosporium species.

In a much preferred embodiment, the kit described herein comprises
(a) at least one mature biofilm of gram-positive microorganisms selected from the group consisting of Staphylococcus (preferably S. aureus, S. epidermidis, S. cohnii), Streptococcus, Enterococcus (preferably E. faecalis), Clostridium, Listeria (preferably L. ivanovii, L. innocua, L. monocytogenes), Micrococcus (preferably M. luteus), Rhodococcus (preferably R. fascians), Cellulosimicrobium, Microbacterium (preferably M. oxydans) and Williamsia (preferably W. muralis) species;
(b) at least one mature biofilm of gram-negative microorganisms selected from the group consisting of Pseudomonas (preferably P. putida, P. fluorescens, P. aeruginosa), Enterobacteriacae (preferably Klebsiella sp., Escherichia coli), Serratia (preferably S. marcescens), Comamonas (preferably C. acidovorans), Bukholderia (preferably B. cepacia), Citrobacter (preferably C. freundii), Campylobacter sp., Leptospira, Legionella (preferably L. pneumophila, L. waltersii, L. Brunensis), Salmonella (preferably S. enterica) and Shewanella (preferably S. putrefaciens) species;
(c) at least one mature biofilm of yeast selected from the group consisting of Rhodotorula (preferably R. mucilaginosa), Cryptococcus, Candida (preferably C. albicans) Saccharomyces and Penicillium species;
(d) at least one positive control; and
(e) at least one negative control.

Said kit is preferably used for the method for describing the efficiency of a biofilm removal treatment described above, more preferably to determine the efficiency of biofilm removal of a washing process in a washing machine or dish washer or for determining the efficiency of any other biocidal treatment.

In order to reliably make the comparisons step (c) of the method described above, it is necessary that biofilms are provided in a standardized manner. Therefore, in another aspect, the invention provides a fixing rail 5 for receiving individual supports 3 for biofilm formation which can be interchangeably fixed in the vessel of a bioreactor 6, preferably in a tank type reactor.

An exemplary embodiment of said fixing rail 5 is depicted in Figs. 2, 3, and 4a to c. The elongated fixing rail 5 typically comprises one or more cavities 10 for receiving and holding the biofilm supports 3. Those cavities 10 may have any form suitable for receiving and holding the supports 3.

In a preferred embodiment, a further aperture 11 is present in the fixing rail 5 which elongates from the cavity 10 towards the opposite side of the fixing rail 5 (see Figs. 2b and 2c). Said aperture 11 is suitable for applying a releasing means for releasing the supports 3 from the fixing rail 5. For example, when the supports 3 are placed within the fixing rail 5, a releasing means shaped like a needle or a device emitting a pressurized gas can be used to push the supports 3 out of the cavities 10.

Within the vessel of a bioreactor 6, said fixing rail 5 is preferably supported in a defined position via a supporting body 7. More preferably, the fixing rail 5 is fixed in a vertical position within the vessel 6. For purposes of handling, e.g. insertion in or removal from the supporting body 7, the fixing rail 5 is preferably equipped with a handling means 15, such as an aperture or a hook like structure.

The supporting body 7 may fix the fixing rails 5 in different ways. Typically, the supporting body 7 is disk- or cylinder-shaped having cavities 16 at its outer border in which the fixing rails 5 may be inserted. An exemplary embodiment of said cavities 16 is shown in Fig. 3. Nevertheless, many other embodiments are known to the skilled person. The supporting body 7 itself can be fixed within the bioreactor 6 in multiple ways. In a preferred embodiment, the supporting body 7 is connected via a holding means 20 to the central axis 21 of the bioreactor 6. In a much preferred embodiment, the surface of the supporting body 7 is flush with the surface of the fixing rails 5.

In a preferred embodiment, the supporting body 7 is connected such that it can turn. For said purpose, the supporting body 7 is connected via the central axis 21 to a motor 22. By spinning the fixing rails 5 connected to the supporting body 7, preferably in a slow manner (e.g. 10 to 500 rpm), the mass transfer of nutrients within the culture broth is enhanced. Furthermore, rotation induces a controlled and uniform shear stress on biofilm 4.

The invention further provides a bioreactor suitable for the production of the biofilms disclosed herein. The arrangement depicted in Fig. 7 is only schematic. It shows a bioreactor 6 which can be of any type, but is preferably a tank reactor 6. Within the bioreactor 6, there is a supporting body 7 (preferably the supporting body described above) which fixes fixing rails 5 in a defined position (three are schematically shown in Fig. 5). The fixing rails 5 are formed such that they can receive and hold supports 3 and are preferably those described herein. Typically, when installed within the bioreactor 6, a number of supports 3 are vertically aligned. The fixing rail 5 comprises means 15 which allows inserting and/or removing the fixing rail 5 from the supporting body 7 during the cultivation cycle via the faceplate 8 of the bioreactor 6. In certain embodiments, more than one supporting body 7 may be present within the bioreactor 6. Further to the supporting body 7 and an adapted faceplate 8, the bioreactor 6 disclosed herein comprises means and/or is connected to means 25 for providing acid and base for pH control, continuously fresh medium and an oxygen loop for aerating the culture.

Ideally, the bioreactor 6 comprises a faceplate 8 with at least one aperture 9b such that one or more fixing rails 5 may me removed, inserted and/or interchanged into the bioreactor 6 during the cultivation cycle. This is preferably done under sterile conditions. Furthermore, the faceplate 8 typically provides means for connecting hoses 13 through which e.g. culture medium from the oxygen loop can be re-introduced into the bioreactor 6. Typically, the faceplate 8 provides different apertures 9, e.g. for connecting the bioreactor to the external environment (9a; e.g. oxygen loop, medium inlet, waste outlet, acid/base for pH control), to insert and remove fixing rails 5 (apertures 9b) or to screw the faceplate to the top of the bioreactor 6 (apertures 9c).

In order to provide standardized biofilm, it is preferable that the culture is not oxygenized via an air sparger. It was surprisingly found that a homogenized biofilm is built over a number of different supports 3 when an oxygen loop is installed. For said oxygen loop, culture broth is taken out of the vessel 6 and mixed with air or oxygen in an aeration bottle 12, e.g. in an adapted Erlenmeyer flask or other recipient 12, and subsequently re-introduced into the vessel 6, all of this preferably under sterile conditions.

In a preferred embodiment, part of the medium from the reactor, preferably about 10% of the culture medium, is continuously circulating in the oxygen loop. Speed and volume of the medium in circulation can be used to adjust the oxygen content in the bioreactor 6.

The air or oxygen is preferably sterilized before it is mixed with the culture medium and is typically provided in a pressure tank or by an aquarium pump 19. One exemplary embodiment is shown in fig. 7. The bioreactor 6 is connected via a hose 13 to an aeration bottle 12. Said aeration bottle 12 is connected via a second hose 13 with the faceplate 8 of the bioreactor 6. The culture broth is transported from the bioreactor 6 into the aeration bottle 12 and from the aeration bottle 12 into the bioreactor 6 via the use of one or more pumps 14. Typically, the faceplate 8 provides one or more connecting means for a hose 13, so that the culture broth can be re-introduced into the bioreactor 6. The aeration bottle 12 is connected to a pressure tank 19 with air, which is flown in a sterile manner into the culture broth in the aeration bottle 12.

In another embodiment, oxygen is introduced into the bioreactor 6 by injection of air or oxygen into the headspace of the bioreactor 6, preferably applying overpressure. At the surface of the culture broth, the gas will enter into the liquid.

In a much preferred embodiment, the bioreactor 6 is equipped for a chemostat culture.

In a preferred embodiment (as depicted in Fig. 7), fresh culture medium for continuous cultivation is added to the aeration bottle 12. Said aeration bottle 12 is connected via hoses 13 to a reservoir 23 such as a medium bag. Within the aeration bottle 12, the medium will be mixed with air or oxygen and subsequently fed to the culture in the bioreactor 6.

Optionally or additionally, medium from the culture is added directly to the bioreactor 6 through faceplate 8.

In a further embodiment, the invention provides a method for providing standardized mature biofilm of microorganisms on a support 3 comprising the steps of
(a) providing one or more supports 3;
(b) inserting the supports 3 into a bioreactor 6, in particular via a fixing rail 5 as described herein in the bioreactor 6 as described herein;
(c) cultivating the microorganisms; and
(d) harvesting the supports 3 with the formed biofilm,
wherein the culture is oxygenized through a oxygen loop.

For the described method, preferably a bioreactor 6 equipped as above is used.

Preferably, the culture is a continuous culture, also called "chemostat culture".

Said oxygen loop, as described above, comprises the steps of
(a) feeding culture broth (i.e. culture medium comprising unattached cells) from the bioreactor 6 to the aeration bottle 12;
(b) optionally adding fresh culture medium to the aeration bottle 12;
(c) aerating said culture broth or the mixture of culture broth and fresh culture medium; and
(d) feeding said aerated culture medium or said mixture into the bioreactor 6.

Figure 10 shows an alternative embodiment of the bioreactor 6 wherein culture broth is added from the bioreactor 6 into the aeration bottle. The supporting body 7 is cylinder shaped and can rotate. For said purpose, the supporting body 7 is connected via the central axis 21 to a motor 22. In the illustration, three fixing rails 5 as shown in Fig. 9 can be seen. In this embodiment, the bioreactor 6 is equipped for continuous cultivation. A medium bag 23 is connected via a hose 13 and a medium inlet 17 to the bioreactor 6. For ensuring continuous cultivation, a medium outlet 18 is provided, as well as pH electrode 27 and an inlet 25 for acid and base to control the pH.

### Examples

### Example 1: Repeatable biofilm formation

Unless otherwise stated the experiments were conducted with biofilm medium containing: 4 g/L glycerol being the carbon source for the microbial growth, 1 g/L 3-Morpholiniumpropanesulfonic acid (MOPS) as buffer, 1.1 g/L Ammonium sulfate((NH₄)2SO₄), 0.15 g/L potassium phosphate monobasic(KH₂PO₄), 0.25 g/L magnesium sulfate heptahydrate (MgSO₄x7H_{2O}), 0.1 g/L iron(II)sulfate heptahydrate(FeSO₄x7H₂O) and 0.2 g/L ethylendiaminetetraacetic acid disodium salt dehydrate (EDTANa₂x2H₂O), 1 ml of trace element solution containing 0.0015 g/L calcium chloride dihydrate (CaCl₂x2H₂O), 0.00396 g/L manganese(II)chloride tetrahydrate(MnCl₂x4H₂O), 0.00562 g/L cobalt sulfate heptahydrate (CoSO₄x7H₂O) 0.00034 g/L copper chloride dihydrate(CuCl₂x2H₂O), 0.001 g/L zinc sulfate heptahydrate(ZnSO₄x7H₂O) and 0.001 g/L sodium molybdate dihydrate(MoO₄Na₂*2H₂O). The pH is adjusted to 7.0 before filter sterilization of the medium. Because this medium is limited such as in Ca and P, it was necessary to supplement with nutrient broth (5g peptone from casein enzymatic digest from Fluka 82303) and 3g meat extract (Fluka 70164)) to get sufficient amounts of cells for inoculation.

### 1a) Production of biofilm from Escherichia coli.

Cells were grown overnight in medium inoculum (30% nutrient broth in biofilm medium supplemented with 4 g per L glycerol). The inoculum was diluted in the bioreactor (dilution 3:50) in the same medium. After 5 hours, the flow of biofilm medium was started. The planktonic cells were first washed out, thereafter the dilution rate was set to 0.1 in order to obtain continuous culture conditions. Coupons were harvested after 1 week. Each coupon was placed in 5 ml of buffer solution (NaCl 9 g/L) and cells were detached by sonication (water bath for 8 min) and vortexing (1 min). Proteins in suspension were quantified using the BCA kit from Sigma (Peterson et al., 1977). Proteins are one of the major constituent of the biofilm and correlate with the increase of biofilm material as it could be measured by optical density a 600nm of the cell suspension.

**Table 1: Amounts of proteins detached from the coupons after one week of continuous cultivation of Escherichia coli in the bioreactor. Results are average ± standard deviation of (n) samples produced during one bioreactor cycle. Extreme values within and between fixing bars are indicated together with number of sample in brackets. Units are mg of proteins equivalent to bovine serum albumin per cm².**

| | 1 Week | |
|---|---|---|
| Proteins | 1.72 ± 0.17 | (17) |
| Variation within fixing bar | 1.64 - 1.96 | (13) |
| Variation between fixing bars | 1.46 - 1.96 | (17) |

### 1b) Production of biofilm from Microbacterium oxydans.

Cells were grown overnight in Tryptic Soy Broth (Fluka 22092) and diluted 1:100 in nutrient broth to constitute the inoculum. The inoculum was diluted in the bioreactor (dilution 3:50) in the biofilm medium containing 30% Nutrient Broth supplemented with 4 g per L glycerol. After 13 hours, the planktonic cells were washed out (dilution rate 0.18 to 0.36). After washing the flow of biofilm medium was reduced to 0.1 in order to obtain continuous culture. Coupons were harvested after 3 days. Each coupon was placed in 5 ml of buffer solution (NaCl 9 g per 1) and cells were detached by sonication (water bath for 8 min) and vortexing (1 min). Proteins in suspension were quantified using the BCA kit from Sigma (Peterson et al., 1977).

**Table 2: Amount of proteins detached from the coupons after 3 days of continuous cultivation of Microbacterium oxydans in the bioreactor. Results are average ± standard deviation of (n) samples produced during one bioreactor cycle. Units are µg of proteins equivalent to bovine serum albumine per cm².**

| | 3 Days | |
|---|---|---|
| Fixing bar A | 64 ± 37 | (18) |
| Fixing bar B | 98 ± 30 | (16) |
| Average experiment | 79 ± 38 | (34) |

### 1c) Production of biofilm from Pseudomonas putida

Cells were grown overnight in medium inoculum (nutrient broth supplemented with 4g per L glycerol). The inoculum was diluted in the bioreactor (dilution 3:50) in the biofilm medium containing 30% Nutrient Broth supplemented with 4g per L glycerol. After 4 to 6 hours, the flow of biofilm medium was started. The planktonic cells were first washed out, thereafter the dilution rate was set to 0.15 in order to obtain continuous culture conditions. Coupons were harvested after 2 weeks. Each coupon was placed in 5 ml of buffer solution (NaCl 9 g per 1) and cells were detached by sonication (US-tip, 10%, 50% active cycle, 1 min) and vortexing (30 s). Proteins in suspension were quantified using the MicroBCA kit from Thermo Scientific (Peterson et al., 1977).

**Table 3: Amount of proteins detached from the coupons after 2 weeks of continuous cultivation of Pseudomonas putida in the bioreactor. Results are average ± standard deviation of (n) samples produced during one bioreactor cycle. Units are µg of proteins equivalent to bovine serum albumin per cm².**

| | 2 Weeks | |
|---|---|---|
| Proteins | 390 ± 130 | (32) |
| Variation within fixing bar | 220 - 540 | (17) |
| Variation between fixing bars | 220 - 620 | (32) |

**Table 4: Amount of proteins detached from the coupons after 2 weeks of continuous cultivation of Pseudomonas putida in the bioreactor. Results are median ± confidence value of 33 samples produced during three independent bioreactor cycle. Units are µg of proteins equivalent to bovine serum albumine per cm².**

| | Proteins | |
|---|---|---|
| Bioprocess 1 | 236 ± 155 | |
| Bioprocess 2 | 422 ± 147 | |
| Bioprocess 3 | 695 ± 262 | |
| Summary of triplicate experiments | 397 ± 39 | |

### 1d) Production of biofilm from Rhodotorula mucilaginosa

Cells were grown overnight in medium inoculum (Sabouraud dextrose broth (SDB), Fluka S3306)). The inoculum was diluted in the bioreactor (dilution 3:50) in the biofilm medium containing 30% SDB. After 8 to 10 hours, the flow of biofilm medium was started. The planktonic cells were first washed out, thereafter the dilution rate was set to 0.12 in order to obtain continuous culture conditions. Coupons were harvested after 1 week. Each coupon was placed in 5 ml of buffer solution (NaCl 9 g per 1) and cells were detached by sonication (US-tip, 10%, 50% active cycle, 1 min) and vortexing (30 s). Proteins in suspension were quantified using the MicroBCA kit from Thermo Scientific (Peterson et al., 1977).

**Table 5: Amount of proteins detached from the coupons after 1 week of continuous cultivation of Rhodotorula mucilaginosa in the bioreactor. Results are average ± standard deviation of (n) samples produced during one bioreactor cycle. Units are µg of proteins equivalent to bovine serum albumine per cm².**

| | 1 Week | |
|---|---|---|
| Proteins | 82 ± 22 | (23) |
| Variation within fixing bar | 59 - 92 | (8) |
| Variation between fixing bars | 59 - 144 | (23) |

**Table 6: Amount of proteins detached from the coupons after 1 week of continuous cultivation of Rhodotorula mucilaginosa in the bioreactor. Results are median ± confidence value of 25 samples produced during three independent bioreactor cycle. Units are µg of proteins equivalent to bovine serum albumine per cm².**

| | Proteins |
|---|---|
| Bioprocess 1 | 74 ± 10 |
| Bioprocess 2 | 76 ± 30 |
| Bioprocess 3 | 80 ± 22 |
| Summary of triplicate experiments | 77 ± 21 |

### Example 2: Biofilm formation on different supports 2a) Pseudomonas putida biofilm on PE, Teflon, metal and PP

The sample holders were loaded with supports of different materials: polyethylene (PE), Teflon, metal and polypropylene (PP). The bioreactor was inoculated with *Pseudomonas putida* to produce biofilms as described above. Coupons were harvested after 2 weeks. Each coupon was placed in 5 ml of buffer solution (NaCl 9 g per 1) and cells were detached by sonication (US-tip, 10%, 50% active cycle, 1 min) and vortexing (30 s).
Polysaccharides in suspension were quantified according to the Dubois method (Dubois, 1956). Polysaccharides are the main constituent of the exopolymeric substances present in biofilm (Characklis and Marshall, 1990) and correlate with the amount of biofilm as can be measured by optical density or crystal violet staining. Proteins in suspension were quantified using the BCA kit from Sigma (Peterson et al., 1977). The number of living cells was quantified by dilution series and plating on Tryptic Soy Agar.

**Table 7: Amount of proteins, polysaccharides and viable cells detached from the different supports after 2 week of continuous cultivation of Pseudomonas putida in the bioreactor. Results are average ± standard deviation of (n=5 samples produced during one bioreactor cycle. Units are µg or cell number per cm².**

| | Proteins | Polysaccharides | Viable cells |
|---|---|---|---|
| PP | 101 ± 59 | 74 ± 25 | 1.8 ± 1.3 10⁷ |
| PE | 82 ± 33 | 141 ± 22 | 2.7 ± 0.9 10⁷ |
| Teflon | 72 ± 45 | 77 ± 31 | 2.7 ± 0.7 10⁷ |
| Metal | 77 ± 40 | 84 ± 46 | 1.0 ± 0.4 10⁷ |

### 2b) Rhodotorula mucilaginosa biofilm on PP supports with different roughness

The sample holders were loaded with support made of PP with different roughnesses. Roughness was created by polishing the smooth PP coupons with coarse and fine sand paper. The bioreactor was inoculated with *Rhodotorula mucilaginosa* to produce biofilms as described above. Coupons were harvested after 1 week. Each coupon was placed in 5 ml of buffer solution (NaCl 9 g per 1) and cells were detached by sonication (US-tip, 10%, 50% active cycle, 1 min) and vortexing (30 s).
Polysaccharides in suspension were quantified according to the Dubois method (Dubois, 1956). Proteins in suspension were quantified using the BCA kit from Sigma (Peterson et al., 1977).

**Table 8: Amount of proteins detached from the different supports after 1 week of continuous cultivation of Rhodotorula mucilaginosa in the bioreactor. Results are median ± standard deviation of (n=5) of samples produced during one bioreactor cycle. Units are µg per cm².**

| | Polysaccharides | Proteins |
|---|---|---|
| smooth | 277 ± 146 | 284 ± 86 |
| Rough- | 704 ± 45 | 399 ± 162 |
| Rough+ | 468 ± 31 | 458 ± 197 |

### Example 3: Storage conditions

### 3a) Storage of Escherichia coli biofilm

Storage tests were conducted on 1-week old *Escherichia coli* biofilm produced as described above. Different storage conditions were tested (Table 9).

**Table 9: Tested storage conditions**

| Code | Temperature (°C) | Glycerol (%) |
|---|---|---|
| a | 25 | none |
| b | 25 | 10 |
| c | 4 | none |
| d | 4 | 10 |
| e | -20 | 10 |
| f | -20 | 50 |
| g | -80 | 10 |
| h | -80 | 50 |

After 11 days and after 2 months the remaining biofilm was evaluated. Each coupon was placed in 5 ml of buffer solution (NaCl 9 g per 1) and cells were detached by sonication (water bath for 8 min) and vortexing (1 min). Polysaccharides in suspension were quantified according to the Dubois method (Dubois, 1956). Proteins in suspension were quantified using the BCA kit from Sigma (Peterson et al., 1977). The number of living cells was quantified by dilution series and plating on Tryptic Soy Agar.

**Table 10: Amounts of living cells, proteins and polysaccharides detached from the coupons colonized with 1-week old Escherichia coli biofilm after 11 days and after 2 months of storage. Results are average ± standard deviation of triplicates. Units are mg or cell number per cm²**

| Storage | Cell numbers | | Proteins | | Polysaccharides | |
|---|---|---|---|---|---|---|
| | Day 11 | Month 2 | Day 11 | Month 2 | Day 11 | Month 2 |
| | | | | | | |
| a | <10⁷ | 4.10³±3.0 | 2.5±1.0 | 2.4±1.0 | nd | 0.4±0.2 |
| b | <10⁷ | 5.10⁴±0.4 | 2.2±0.8 | 1.9±0.5 | nd | 0.2±0.0 |
| c | 8.10⁹±1.0 | 5.10⁷±4.7 | 2.7±0.4 | 2.2±0.3 | nd | 0.5±0.1 |
| d | 4.10⁹±0.4 | 6.10⁷±1.0 | 2.5±0.6 | 2.0±0.5 | nd | 0.4±0.1 |
| e | nd | nd | 0.5±0.3 | nd | nd | nd |
| f | nd | nd | 0.6±0.4 | nd | nd | nd |
| g | nd | nd | 0.6±0.3 | nd | nd | nd |
| h | nd | nd | 0.8±0.5 | nd | nd | nd |

The best conservation of *Escherichia coli* biofilms was obtained at 4°C. At this temperature, the addition of glycerol did not significantly improve the conservation.

### 3b) Storage of Pseudomonas putida biofilm

Storage tests were conducted on 2-week old *Pseudomonas putida* biofilm produced as described above. Different storage conditions were tested (Table 11).

**Table 11: Tested storage conditions**

| Code | Temperature (°C) | Protectant (conc) |
|---|---|---|
| a | -20 | Trehalose (0.1M) |
| b | -20 | Hydroxyectoine (0.4M) |
| c | -20 | TE buffer |
| d | -20 | Glycerol (10%) |
| e | -20 | none |
| f | 4 | Trehalose (0.1M) |
| g | 4 | Hydroxyectoine (0.4M) |
| h | 4 | TE buffer |
| i | 4 | Glycerol (10%) |
| j | 4 | none |
| k | 20 | Trehalose (0.1M) |
| l | 20 | Hydroxyectoine (0.4M) |
| m | 20 | TE buffer |
| n | 20 | Glycerol (10%) |
| o | 20 | none |

After 11 days and after 2 months the remaining biofilm was evaluated. Each coupon was placed in 5 ml of buffer solution (NaCl 9 g per 1) and cells were detached by sonication (US-tip, 10%, 50% active cycle, 1 min) and vortexing (30 s). Polysaccharides in suspension were quantified according to the Dubois method (Dubois, 1956). Proteins in suspension were quantified using the MicroBCA kit from Thermo Scientific(Peterson et al., 1977). The number of living cells was quantified by dilution series and plating on Tryptic Soy Agar. Before storage the biofilm contained per cm² 0.40 mg proteins, 0.20 mg polysaccharides and 1 10⁹ cells.

**Table 12: Amounts of living cells, proteins and polysaccharides detached from the coupons colonized with 2-week old Pseudomonas putida biofilm after 11 days and after 2 months of storage. Results are average of triplicates. Units are mg or cell number per cm².**

| Storage | Cell numbers | | Proteins | | Polysaccharides | |
|---|---|---|---|---|---|---|
| | Day 11 | Month 2 | Day 11 | Month 2 | Day 11 | Month 2 |
| | | | | | | |
| a | 1.5 10⁷ | nd | 0.10 | nd | nd | nd |
| b | < 1 10¹ | nd | 0.07 | nd | 0.16 | nd |
| C | < 1 10¹ | nd | 0.02 | nd | 0.02 | nd |
| d | < 1 10¹ | nd | 0.05 | nd | nd | nd |
| e | < 1 10¹ | nd | 0.11 | nd | nd | nd |
| f | 2.2 10⁸ | 7.4 10⁶ | 0.22 | 0.14 | nd | nd |
| g | 9.9 10⁸ | 2.1 10⁸ | 0.33 | 0.23 | 0.35 | 0.08 |
| h | <1 10¹ | < 1 10¹ | 0.03 | 0.05 | 0.05 | 0.06 |
| i | 2.8 10⁸ | 2.5 10⁸ | 0.32 | 0.20 | 0.12 | 0.11 |
| j | 5.7 10⁷ | nd | 0.36 | 0.30 | 0.14 | 0.12 |
| k | 3.3 10⁷ | 1.2 10⁶ | 0.25 | 0.23 | nd | nd |
| l | 6.7 10⁶ | 6.4 10⁹ | 0.30 | 0.24 | 0.10 | 0.11 |
| n | 1 10⁷ | < 1 10¹ | 0.21 | 0.16 | 0.08 | 0.08 |
| o | 1.3 10⁷ | 1.3 10⁷ | 0.32 | 0.31 | 0.11 | 0.12 |

The best conservation of *Pseudomonas putida* biofilms was obtained at 4°C in presence of 0.4M hydroxyectoine. Under this condition less than 1 log of living cells was lost over a 2-months period of storage.

### 3c) Storage of Rhodotorula mucilaginosa biofilm

Storage tests were conducted on 1-week old *Rhodotorula mucilaginosa* biofilm produced as described above. Different storage conditions were tested (Table 13).

**Table 13: Tested storage conditions**

| Code | Temperature | Protectant |
|---|---|---|
| | (°C) | (conc) |
| a | -20 | Trehalose (0.1M) |
| b | -20 | Hydroxyectoine (0.4M) |
| c | -20 | TE buffer |
| d | -20 | none |
| e | 4 | Trehalose (0.1M) |
| f | 4 | Hydroxyectoine (0.4M) |
| g | 4 | TE buffer |
| h | 4 | none |
| i | 20 | Trehalose (0.1M) |
| j | 20 | Hydroxyectoine (0.4M) |
| k | 20 | TE buffer |
| l | 20 | none |

After 11 days the remaining biofilm was evaluated. Each coupon was placed in 5 ml of buffer solution (NaCl 9 g per 1) and cells were detached by sonication (US-tip, 10%, 50% active cycle, 1 min) and vortexing (1 min). Polysaccharides in suspension were quantified according to the Dubois method (Dubois, 1956). Proteins in suspension were quantified using the MicroBCA kit from Thermo Scientific (Peterson et al., 1977). The amount of living cells was determined by dilution series and plating on Sabouraud Dextrose Agar. Before storage the biofilm contained per cm² 82 µg proteins, 359 µg polysaccharides and 3.1 10⁸ cells.

**Table 14: Amounts of living cells, proteins and polysaccharides detached from the coupons colonized with 1-week old Rhodotorula mucilaginosa biofilm after 11 days of storage. Results are average ± standard deviation of triplicate measurements. Units are µg or cell number per cm².**

| Storage | Cell numbers | Proteins | Polysaccharides |
|---|---|---|---|
| a | 5.3 ± 3.7 10⁶ | 138 ± 60 | nd |
| b | 9.0 ± 4.6 10⁶ | 151 ± 73 | 1476 ± 746 |
| c | 5.7 ± 5.6 10⁶ | 57 ± 31 | 584±316 |
| d | 3.3 ± 1.5 10⁷ | 284 ± 99 | 2902 ± 1011 |
| e | 1.3 ± 0.1 10⁷ | 225 ± 37 | nd |
| f | 1.3 ± 0.5 10⁷ | 150 ± 23 | 1530 ± 238 |
| g | 5.8 ± 3.8 10⁶ | 131 ± 37 | 1341 ± 379 |
| h | 1.1 ± 1.0 10⁷ | 181 ± 28 | 1842± 282 |
| i | 8.3 ± 4.6 10⁶ | 462 ± 46 | nd |
| j | 7.5 ± 3.2 10⁶ | 110 ± 43 | 1120 ± 439 |
| k | 1.9 ± 1.5 10⁶ | 96 ± 25 | 979 ± 2 5 7 |
| 1 | 1.7 ± 0.6 10⁷ | 162 ± 27 | 1652 ± 277 |

After 11 days storage the higher recovery of *Rhodotorula mucilaginosa* was obtained for biofilm stored at -20°C without protectants.

### Example 4: Washing test

Washing tests were conducted on 1-week old *P*. *putida* biofilm. Biofilm was produced as describe above. Before washing test the biofilm were stored for 2 weeks at 4°C in presence of trehalose as describe above.

Washing tests were conducted at 30°C with standard formulations of liquid and powder detergents. Carriers containing *Pseudomonas putida* biofilm and a negative control (coupon without biofilm) were placed at 3 locations within the household washing machines (right, left, and back)

Before and after washing remaining biofilm was evaluated. Each coupon was placed in 5 ml of buffer solution (NaCl 9 g per 1) and cells were detached by sonication (US-tip, 10%, 50% active cycle, 1 min) and vortexing (30 s). Proteins in suspension were quantified using the microBCA kit from Thermo Scientific. Cell numbers was determined by dilution serie and plating on Tryptic Soy Agar. Cell activity was determined via measurement of pH changes using the Solaris method (Neogen). Total amount of biofilm was quantified by direct staining with Crystal Violet (final concentration of 0.1%). Color intensity was determined with a colorimeter (Minolta, CM-2600d) according to Lab color chart. Color measurements are reported after substration of color values of blank coupons.

**Table 15: Amounts of living cells, proteins and polysaccharides detached from the coupons colonized with 1-week old Pseudomonas putida biofilm, and color intensity after crystal violet staining. Coupons were evaluated just after harvesting (fresh), after 11 days of storage and after washing process. Results are average ± standard deviation of triplicate measurements. Units are µg or cell number per cm² or difference of color intensity expressed according to the Lab scale.**

| Sample | Cell | Activity | Proteins | Crystal |
|---|---|---|---|---|
| | numbers | | | violet |
| | | | | (ΔL, Δa, Δb) |
| Fresh | 1.2 10⁷ | | 87 | 44, -31, 29 |
| After storage | 1.4 10⁷ | > 5 10⁶ | 62 | 42, -32, 35 |

| After washing with liquid detergent | | | | |
|---|---|---|---|---|
| Location right | 1.8 10⁶ | > 5 10⁶ | 35 | 36, -26, 33 |
| Location left | 5.3 10⁵ | > 5 10⁶ | 37 | 29, -20, 29 |
| Location back | 2.3 10⁵ | 5 10⁴ - | 56 | 39, -32, 38 |
| | | 5 10⁶ | | |

| After washing with powder detergent | | | | |
|---|---|---|---|---|
| Location right | 1.4 10⁵ | 5 10⁴ - | 32 | 7, -5, 9 |
| | | 5 10⁶ | | |
| Location left | 2.0 10³ | < 5 10² | 30 | 7, -6, 10 |
| Location back | 2.7 10⁶ | 5 10⁴ - | 78 | 50, -36, 34 |
| | | 5 10⁶ | | |

The biofilm kit allowed to differentiate the washing efficiency of the 2 type od detergents, and confirmed previous findings showing that liquid detergents are less efficient against biofilm than powder detergent. The washing efficiency varies according to location within the washing machine. The maximum of biofilm removal was observed on the left side of the washing machine.

While there are shown and described presently preferred embodiments of the invention, it is to be distinctly understood that the invention is not limited thereto but may be otherwise variously embodied and practiced within the scope of the following claims.

### References

Characklis W.G. and Marshall K.C. (1990) Biofilms. Wiley eds, 796 pp.
Dubois M., Gilles K.A., Hamilton J.K., Rebers P.A., and Smith F. (1956) Colorimetric method for determination of sugars and related substances. Analytical Cemisty 28: 350-356.
Gjaltema A., Arts P.A.M., Vanloosdrecht M.C.M., Kuenen J.G. and Heijnene J.J. (1994) Heterogeneity of biofilms in rotating annular reactors - occurrence, structure, and consequences. Biotechnology and Bioengineering 44: 194-204.
Kharazmi A., Giwercman B. and Hoiby N. (1999) Robbins device in biofilm research. Methods in Enzymology - Biofilms 310: 207-215.
Peterson G.L.(1977) Simplification of protein assay method of Lowry et al., which is more generally applicable. Analytical biochemistry 83: 346-356.
Peyton B.M. and Characklis W.G. (1993) A statistical-analysis of the effect of substrate utilization and shear-stess on the kinetic of biofilm detachment. Biotechnology and Bioengineering 41: 728-735.
Millar M.R., Linton C.J. and Sheriff A. (2001) Use of continuous culture system linked to a modified Robbins device or flow cell to study attachment of bacteria to surfaces. Methods in enzymology - Microbial growth in biofilms 337: 43-62.

### Reference numbers:

- Sample device: 1
- Carrier structure: 2
- supports or coupons: 3
- biofilm: 4
- fixing rail: 5
- bioreactor: 6
- supporting body: 7
- faceplate: 8
- apertures in faceplate: 9
- cavities in fixing rail: 10.
- aperture in fixing rail: 11
- aeration bottle: 12
- hose: 13
- pump: 14
- aperture in fixing rail: 15
- cavity in supporting body: 16
- medium inlet: 17
- medium outlet: 18
- gas bottle: 19
- holding means: 20
- central axe: 21
- motor: 22
- medium bag: 23
- color chart: 24
- acid base reservoir for pH control: 25
- screw: 26
- pH electrode: 27

## Claims

1. A mature in vitro biofilm of microorganisms on a support; in particular, wherein said biofilm is
(i) standardized;
(ii) has a specific strain composition; and/or
(iii) is reproducible and/or repeatable.

2. The biofilm of claim 1, obtainable by a method comprising the steps of
(a) providing one or more supports (3);
(b) providing an inoculum of one or more microorganisms;
(c) inserting the supports (3) into a bioreactor (6);
(d) cultivating the microorganisms in batch, fed-batch or continuous mode;
(e) aerating the culture via an oxgygen loop; and
(f) harvesting the biofilm formed on the supports.

3. The biofilm of claim 1 or 2, wherein the oxygen loop comprises the steps of
(a) optionally adding fresh culture medium to the aeration bottle (12);
(b) adding culture broth of the culture to the aeration bottle (12);
(c) aerating said culture broth or the mixture of culture broth and fresh culture medium; and
(d) feeding said aerated culture broth or said mixture into the bioreactor (6).

4. The biofilm of claim 1 to 3, wherein the biofilm(s) consist of microorganisms of a single species or of several species, selected from the group consisting of Pseudomonas (in particular P. putida, P. fluorescens), Staphylococcus (in particular S. cohnii), Enterobacteriacae (in particular Escherichia coli and Klebsiella), Micrococcus (in particular M. luteus, M. oxydans), Rhodotorula (in particular R. mucilaginosa, R. slooffiae), Rhodococcus sp., Cellulosimicrobium, Candida, Citrobacter (in particular C. freundii) and Microbacterium species.

5. A method for determining the efficiency of a biofilm removal treatment comprising:
(a) providing at least one support (3) with a standardized mature biofilm;
(b) subjecting the support(s) (3) and the biofilm(s) to a treatment to be assessed and evaluated; and
(c) determining the degree of removal of the biofilm(s) from the support (3) by comparing to a untreated biofilm(s).

6. The method of claim 5 wherein the biofilm(s) of step a) are the standardized mature biofilm of claims 1 to 4,

7. The method of claim 5 or 6, wherein the treatment is washing, cleaning, disinfecting, sterilizing or combinations thereof.

8. The method of anyone of claims 5 to 7, wherein step (c) is performed by
(i) staining the biofilm and comparing the treated and the un-treated biofilm(s) with a color scale,
(ii) quantitative PCR; and/or by
(iii) detecting a biofilm specific indicator.

9. A kit for determining the efficiency of a biofilm removal treatment by the method of anyone of claims 5 to 7, comprising a sample device (1), said sample device comprising a carrier (2), said carrier (2) comprising one or more supports (3) with a mature standardized biofilm.

10. The kit of claim 9 wherein the mature biofilm is the biofilm of anyone of claim 1 to 4.

11. The kit of claim 9 or 10, further comprising storage and/or transporting means for the carrier and/or an evaluation device.

12. The kit of claim 11, wherein the evaluation device comprises agents for staining the biofilm and a color scale.

13. The kit of anyone of the claims 9 to 12, comprising several biofilms on distinct supports (3), each biofilm consisting of a single species or of several species of microorganisms.

14. A fixing rail (5) comprising one or more cavities (10) for receiving individual supports (3) for biofilm formation which can be interchangeably fixed in the vessel of a bioreactor (6), in particular a tank reactor.

15. A bioreactor (6) with a faceplate (8) for the production of mature standardized biofilm, comprising
(i) a supporting body (7) to support the fixing rail (5) of claim 14 in a defined position in the vessel (6);
(ii) one or more apertures (9) in said faceplate (8) to remove or interchange said fixing rails,
and
(iii) further comprising means or being connected to means for an oxygen loop.
